# EUROPÄISCHE PATENTSCHRIFT

(11) **EP 0 427 162 B1**
(45) Veröffentlichungstag und Bekanntmachung des Hinweises auf die Patenterteilung: **08.05.1996**
(21) Anmeldenummer: 90121101.1
(22) Anmeldetag: 03.11.1990
(51) Int. Cl.: C07K 14/62, A61K 38/28, C12P 21/02

(54) **Neue Insulinderivate, Verfahren zu deren Herstellung, ihre Verwendung und eine sie enthaltende pharmazeutische Zubereitung**
Novel insulin derivatives, their preparation and use, and a pharmaceutical composition thereof
Nouveaux dérivés de l'insuline, leur préparation, leur usage et une composition pharmaceutique les contenant

(30) Priorität: 06.11.1989 DE 3936876
(43) Veröffentlichungstag der Anmeldung: 15.05.1991
(73) Patentinhaber: HOECHST AKTIENGESELLSCHAFT, D-65926 Frankfurt am Main (DE)
(72) Erfinder: Vértesy, László, Dr., W-6239 Eppstein/Taunus (DE); Geisen, Karl, Dr., W-6000 Frankfurt am Main (DE); Bicker, Richard, Dr., W-6237 Liederbach (DE)

(56) Entgegenhaltungen:
- EP-A- 0 140 084
- PROCEEDINGS OF THE FIFTH AMERICAN PEPTIDE SYMPOSIUM, ed. Goodman et al., 1977, Seiten 136-140, Wiley, New York, US; H.-J. FRIESEN et al.: "Structure-function relationships of insulins modified in the A1-region"
- BIOCHEM. JOURNAL, Band 186, 1980, Seiten 945-952, London, GB; P. RÖSEN et al.:
- "Binding of insulin to bovine liver plasma membrane"

## Beschreibung

Insulin und Insulinderivate werden bekanntlich in erheblichen Mengen zur Behandlung der Krankheit Diabetes mellitus benötigt und z.T. auch großtechnisch produziert. Trotz der beträchtlichen Zahl der bereits existierenden Insulin-Zubereitungen und -Abwandlungen mit unterschiedlichen Wirkungsprofilen besteht wegen der Verschiedenheit der Organismen mit deren inter- und intraindividuellen Schwankungen immer noch ein Bedarf an weiteren Insulinprodukten mit wieder anderen Eigenschaften und Wirkungscharakteristiken.

Insulinderivate mit einer verzögerten Wirkung sind z.B. beschrieben in EP-B-132 769 und EP-B-132 770. Es handelt sich um speziell in der Position B31 der Insulin-B-Kette basisch modifizierte Derivate der folgenden Formel I: 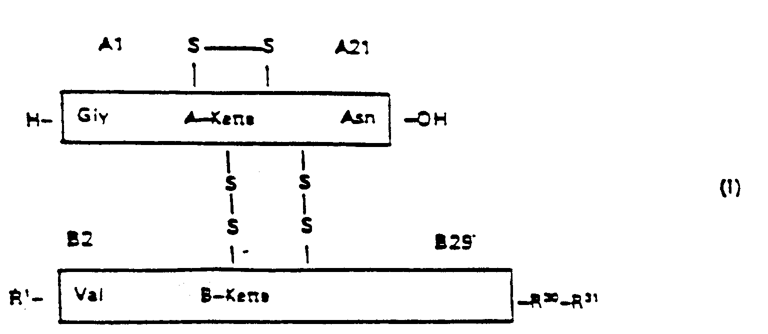
in welcher R¹ H oder H-Phe bedeutet,
R³⁰ für den Rest einer neutralen, genetisch kodierbaren L-Aminosäure und
R³¹ für eine physiologisch unbedenkliche organische Gruppe basischen Charakters mit bis zu 50 C-Atomen steht, an deren Aufbau 0 bis 3 α-Aminosäuren beteiligt sind und deren gegebenenfalls vorhandene endständige Carboxylfunktion frei, als Esterfunktion, als Amidfunktion, als Lacton oder zu CH₂OH reduziert vorliegen kann.

Für diese Insulinderivate ist ein isoelektrischer Punkt zwischen 5,8 und 8,5 (gemessen in der isoelektrischen Fokussierung) charakteristisch. Der - gegenüber dem isoelektrischen Punkt des unmodifizierten nativen Insulins oder Proinsulins (bei pH = 5,4) in den Neutralbereich herein verschobene - isoelektrische Punkt ist durch die zusätzliche(n), an der Oberfläche des Moleküls befindliche(n) positive(n) Ladung(en) infolge der basischen Modifikation bedingt. Damit sind diese basisch modifizierten Insulinderivate im physiologisch wichtigen Neutralbereich weniger löslich als etwa natives Insulin oder Proinsulin, welche im Neutralbereich normalerweise gelöst vorliegen.

Die Verzögerungs- oder Depot-wirkung der basisch modifizierten Insulinderivate der Formel I geht auf deren Schwerlöslichkeit am isoelektrischen Punkt zurück. Die Wiederauflösung der Insulinderivate unter physiologischen Bedingungen soll nach den beiden vorerwähnten Druckschriften durch Abspaltung der zusätzlichen basischen Gruppen erreicht werden, was je nach Derivat durch tryptische oder Trypsin-ähnliche und/oder Carboxypeptidase B oder der Carboxypeptidase B-ähnliche und/oder Esterase-Aktivität zustande kommt. Die jeweils abgespaltenen Gruppen sind entweder rein physiologische Metaboliten oder aber leicht metabolisierbare, physiologisch unbedenkliche Substanzen.

Das vorerwähnte Depotprinzip infolge basischer Modifikation des Insulins wurde in der Folgezeit weiter genutzt durch die Bereitstellung und entsprechende Verwendung anderer - hauptsächlich innerhalb der A- und B-Ketten - basisch modifizierter Insulinderivate; vgl. z.B. EP-A-0194 864 und EP-A-0254 516.

In dem Dokument Proc. Am. Peptide Symp (1977), Seiten 136-140, werden bereits an A1 modifizierte Rinderinsuline beschrieben.

Es sind auch einige basisch modifizierte Insulinderivate bekannt, bei denen sich die basische Modifizierung in der Verlängerung der A-Kette über die Position A1 hinaus befindet; vgl. P. Rösen et al., Biochem. J. (1980) Bd. 186, 945 - 952. Als solche Insulinderivate mit basischen Aminosäuren als Modifizierungskomponenten sind in dieser Literaturstelle speziell beschrieben:
Lys-Arg-Gly^{A1}-Rinderinsulin
Arg-Gly^{A1}-Rinderinsulin,
Arg-Arg-Gly^{A1}-Rinderinsulin und
Arg-Arg-Arg-Gly^{A1}-Rinderinsulin.

Diese Insulinderivate sollen gegenüber unmodifiziertem Insulin eine erheblich verringerte biologische Aktivität besitzen; vgl. insbesondere Tab. 1 auf S. 947 der genannten Literaturstelle. Über eine etwaige Depot-Wirkung der Verbindungen ist in der Literaturstelle nichts ausgesagt.

Besonders vorteilhafte, basisch modifizierte Insulinderivate mit Depot-Wirkung stellen die basisch modifizierten Insulinderivate gemäß der DE-Patentanmeldung DE 38 44 211 vom 29.12.1988 dar; es handelt sich um Insulinderivate der nachstehenden Formel II, an deren A0-Position die basische Aminosäure Arginin Sitzt: 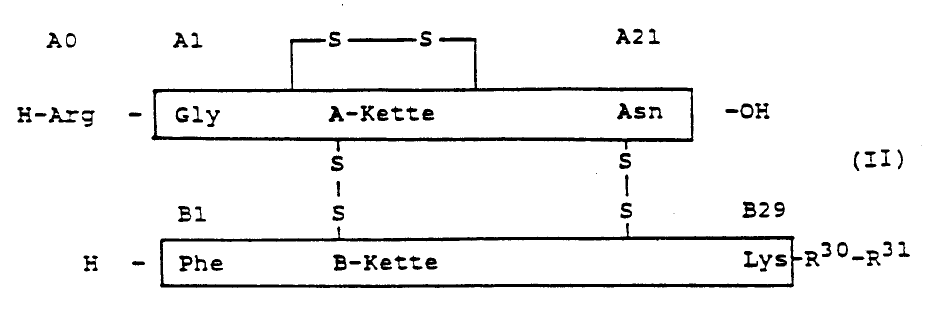
in welcher
a)
   - R³⁰ + R³¹ zusammen =: OH oder
b)
   - R³⁰ =: Rest einer neutralen, genetisch kodierbaren L-Aminosäure und
   - R³¹ =: OH oder
   eine physiologisch unbedenkliche organische Gruppe basischen Charakters mit bis zu 50 C-Atomen, an deren Aufbau 0 bis 3 α-Aminosäuren beteiligt sind und deren gegebenenfalls vorhandene endständige Carboxylfunktion frei, als Esterfunktion, als Amidfunktion, als Lacton oder zu CH₂OH reduziert vorliegen kann,
mit Ausnahme des Falles, in welchem gleichzeitig R³⁰ = Ala, R³¹ = OH sowie die A- und B-Kette die Sequenzen des Rinderinsulins sind (d. i. also AO-Arg-Rinderinsulin).

Die physiologisch verträglichen Salze (wie z.B. die Alkali- oder Ammoniumsalze) dieser Insulinderivate sind den freien Insulinderivaten äquivalent.

Diese Insulinderivate weisen infolge ihrer basischen Modifikation in A0-Position - wie auch die vorher erwähnten basisch modifizierten Insulinderivate - ein verzögertes Wirkungsprofil und - gegenüber den vorher erwähnten basisch modifizierten Insulinderivaten - deutliche Vorteile in Bezug auf die Verträglichkeit im Organismus auf; ihre biologische Aktivität entspricht derjenigen von nativem Insulin.

In dem Bestreben, diese Insulinderivate noch weiterzuentwickeln, wurde nun gefunden, daß dieses Ziel durch eine Verlängerung der A-Kette über die AO-Arg-Position hinaus gelingt.

Erfindungsgegenstand sind daher neue Insulinderivate der Formel III 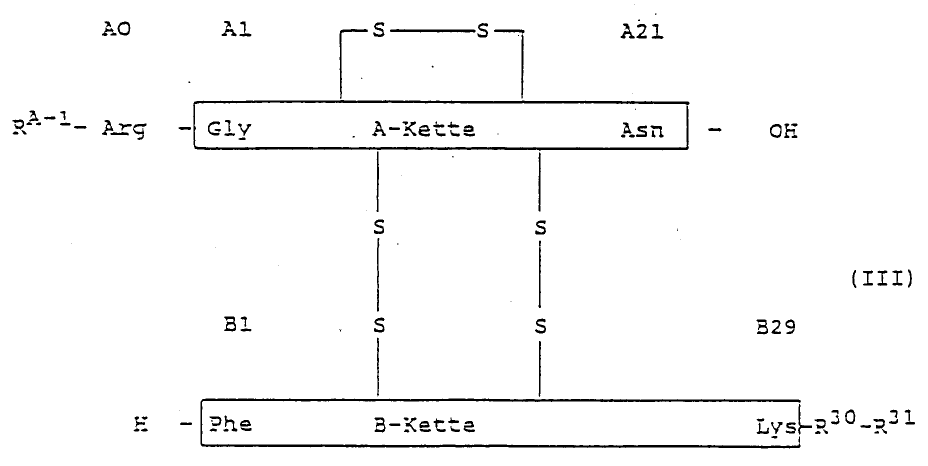
in welcher
a)
   - R³⁰ und R³¹: zusammen OH bedeuten oder
b)
   - R³⁰: bedeutet Rest einer neutralen, genetisch kodierbaren L-Aminosäure und
   - R³¹: bedeutet OH oder 1 bis 3 α-Aminosäuren, und
c)
   - R^{A-1}: bedeutet Rest einer genetisch kodierbaren L-Aminosäure aus der Gruppe Thr, Ser oder Ala; sowie physiologisch verträgliche Salze des Insulinderivats der Formel III,

a) Die Verbindungen der Formel III mit R³⁰ + R³¹ zusammen = OH sind die entsprechenden R^{A-1}-A0-Arg-Des-B30-Insuline; diese Verbindungen sind besonders bevorzugt.
b) Alternativ kann in Formel III R³⁰ auch der Rest einer neutralen, genetisch kodierbaren L-Aminosäure und
   R³¹ = OH oder
   eine entsprechende physiologisch unbedenkliche organische Gruppe basischen Charakters mit bis zu 50 C-Atomen sein; diese Verbindungen sind entsprechende R^{A-1}-AO-Arg-Insulinderivate.

R³⁰ und R³¹ besitzen die gleiche Bedeutung wie in Formel II für die Verbindungen gemäß der vorerwahnten älteren Patentanmeldung.

Neutrale, genetisch kodierbare L-Aminosäuren - für R³⁰ - sind Gly, Ala, Ser, Thr, Val, Leu, Ile, Asn, Gln, Met, Tyr, Phe und Pro; bevorzugt sind hierunter Ala, Thr und Ser, insbesondere nur Thr.

Falls R³¹ = OH, resultieren Insulinderivate, welche sich von den entsprechenden Insulinen nur durch die Modifikation in (A-1)- und A0-Position unterscheiden.

Wenn am Aufbau von R³¹ bis Zu 3 α-Aminosäuren beteiligt sind, sind dies in erster Linie neutrale oder basische natürlich vorkommende L-Aminosäuren und/oder die diesen entsprechenden D-Aminosäuren. Neutrale natürlich vorkommende Aminosäuren sind insbesondere Gly, Ala, Ser, Thr, Val, Leu, Ile, Asn, Gln, Cys, Met, Tyr, Phe, Pro und Hyp. Basische natürlich vorkommende Aminosäuren sind insbesondere Arg, Lys, Hyl, Orn, Cit und His.

Für R^{A-1} kann der Rest jeder der genetisch kodierbaren Aminosäuren aus der Gruppe Ala, Thr oder Ser, insbesondere nur Thr, stehen.

Bevorzugte Insulinderivate der Formel III sind diejenigen mit
a)
   - R³⁰ + R³¹ =: OH oder
b)
   - R³⁰ =: Rest von Ala, Thr oder Ser, insbesondere von Thr, und
   - R³¹ =: OH, sowie
c)
   - R^{A-1} =: Thr.

Diese Insulinderivate sind (A-1)Thr-(AO)Arg-Des(B30)-Insuline sowie (A-1)Thr-(AO)Arg-Insuline.

Wenn hier die A1 bis A21- sowie die B1 bis B29-Sequenzen die (identischen) Sequenzen der Human-, Schweine- oder Kanincheninsulins oder die (darin geringfügig verschiedenen) Sequenzen des Rinderinsulins sind, handelt es sich um (A-1)Thr(AO)Arg-Des-(B30)-Human-, -Schweine-, -Kaninchen- oder -Rinderinsulin, sowie um (A-1)Thr-(AO)Arg-Humaninsulin [mit (B30)Thr], um (A-1)Thr-(AO)Arg-Schweineinsulin [mit (B30)Ala], um (A-1)Thr-(AO)Arg-Kanincheninsulin [mit (B30)Ser] und um (A-1)Thr-(AO)Arg-Rinderinsulin [mit (B30)Ala].

Besonders bevorzugte Insulinderivate der Formel III sind diejenigen mit
a)
   - R³⁰ + R³¹ =: OH und
b)
   - R^{A-1} =: Thr,
also die (A-1)Thr-(AO)Arg-Des-(B30)-Insuline, insbesondere das (A-1)Thr-(AO)Arg-Des(B30)-Humaninsulin.

Die A-Kette und die B1- bis B29-Kette in Formel III können im Prinzip die Sequenzen aller möglichen Insuline sein; vorzugsweise sind sie jedoch die Sequenzen des Human-, Schweine-, Kaninchen- oder Rinder-Insulins, insbesondere die Sequenzen des Humaninsulins (welche mit den A1- bis A21- und B1- bis B29-Sequenzen des Schweine- und Kanincheninsulins identisch sind).

Der isoelektrische Punkt der Insulinderivate der Formel III liegt zwischen 5,5 und 9,0 (gemessen in der isoelektrischen Fokussierung).

Die Herstellung der Insulinderivate der Formel III kann dadurch erfolgen, daß man
a) ein Insulinderivat der Formel IV 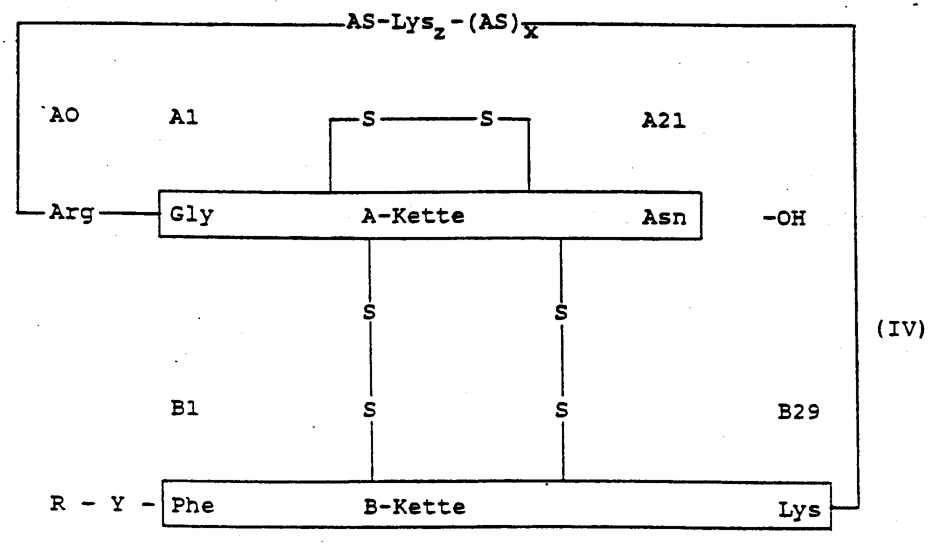 worin
   - AS =: genetisch kodierbare Aminosäure(n),
   - x,z =: unabhängig voneinander 0 oder ganze Zahlen von 1 - 50, wobei allerdings - falls x ≠ 0 - auch z ≠ 0 ist,
   - R =: organischer Rest mit bis zu 50 C-Atomen, vorzugsweise ein Rest einer genetisch kodierbaren Aminosäure oder ein Peptidrest aus zwei oder mehreren gleichen oder verschiedenen genetisch kodierbaren Aminosäuren,
   - y =: Lys oder Arg,
   und die A- und B(1 bis 29)-Ketten vorzugsweise die Sequenzen des Human-, Schweine-, Kaninchen- oder Rinderinsulins, insbesondere des Human-, Schweine- oder Kanincheninsulins, aufweisen,
   mit Lysylendopeptidase in Kontakt bringt, wobei die Bindungen am C-terminalen Ende der Lysylreste gespalten werden, und - falls Y = Lys - die Verbindungen der Formel III mit R³⁰ + R³¹ zusammen = OH und R^{A-1} = genetisch kodierbare Aminosäure erhalten werden, bzw. - falls Y = Arg - noch mit Trypsin oder einer trypsinähnlichen Endopeptidase versetzt wird, wobei die Präaminosäuresequenz R-Arg von der B-Kette abgespalten wird und ebenfalls eine Verbindung der Formel III mit R³⁰ + R³¹ zusammen = OH und R^{A-1} = genetisch kodierbare Aminosäure entsteht, oder daß man
b) ein ggfs. geschütztes Insulinprodukt der Formel V 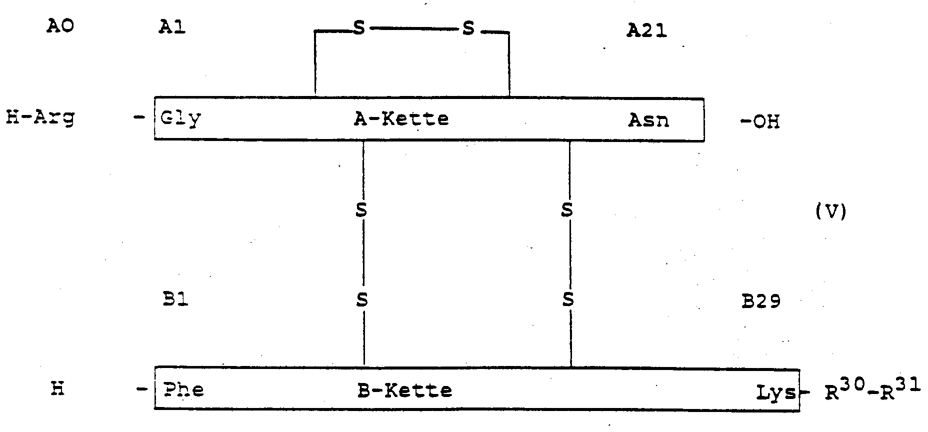 in welcher
   R³⁰ und R³¹ die gleiche Bedeutung wie in Formel III besitzen,
   am N-Terminus des (AO)-Arginins in an sich bekannter Weise mit ggfs. geschützten und/oder aktivierten, genetisch kodierbaren L-Aminosäuren, oder mit Donoren von physiologisch unbedenklichen organischen Resten mit bis zu 50 C-Atomen, vorzugsweise von - ggfs. substituierten - Alkyl-, Aryl- oder Alkarylresten mit nicht über 50 C-Atomen umsetzt und anschließend die vorhandenen Schutzgruppen in bekannter Weise abspaltet.

Die Ausgangsprodukte der Verfahrensvariante a) - Die Insulinvorprodukte der Formel IV - werden vorzugsweise nach gentechnologischen Methoden gewonnen, wie sie z. B. beschrieben sind in den DE-Patentanmeldungen DE 38 21 159 (vom 23.06.1988) und DE 38 37 273 (vom 03.11.1988) sowie der bereits vorher erwähnten DE-Patentanmeldung DE 38 44 211 (vom 29.11.1988).

Falls in den Insulinvorprodukten der Formel IV Y = Lys, verläuft die enzymatische Spaltung mittels Lysylendopeptidase direkt bis zu den entsprechenden Des-B30-Endprodukten der Formel III (mit R³⁰ + R³¹ zusammen = OH, und R^{A-1} = genetisch kodierbare Aminosäure); falls Y = Arg, verbleibt bei der Spaltung mittels Lysylendopeptidase am N-terminalen Ende der B-Kette noch der Rest R-Y (weil Lysylendopeptidase Peptidketten nur an der Carboxyseite von Lys spaltet). Dieser Rest wird dann vorzugsweise noch mittels Trypsin abgespalten, wobei ein Substrat/Enzym-Verhältnis von etwa (100 - 100.000) : 1, insbesondere von etwa (1.000 - 10.000) : 1 zweckmäßig ist. Es resultiert das gleiche Endprodukt wie in dem Fall des Ausgangsprodukts mit Y = Lys. Ansonsten werden die enzymatischen Spaltungen in an sich bekannter Weise und vorzugsweise bei Raumtemperatur durchgeführt.

Die Ausgangsprodukte für Verfahrensvariante b) - die Insulinprodukte der Formel V - sind die in der Patentanmeldung DE 38 44 211 (vom 29.11.1988) beschriebenen AO-Arg-Insulinderivate. Sie können für den Einsatz in Verfahrensvariante b) noch mit - in der Peptidchemie üblichen - Schutzgruppen für die Amino- und/oder Carboxylgruppen versehen werden, wobei natürlich der N-Terminus des AO-Arginins ungeschützt bleiben muß (weil dort die Anknüpfung des Restes R^{A-1} erfolgen muß). Die - ggfs. entsprechend geschützten - Insulinprodukte der Formel V werden dann mit - ebenfalls ggfs. geschützten und/oder aktivierten - genetisch kodierbaren Aminosäuren oder mit Donoren von physiologisch unbedenklichen organischen Resten mit bis zu 50 C-Atomen, vorzugsweise von - ggfs. substituierten - Alkyl-, Aryl- oder Aralkylresten (z. B. Alkyl-, Aryl- oder Aralkylhalogeniden) mit bis zu 50 C-Atomen in für derartige Reaktionen üblicher Weise umgesetzt; einige in Frage kommende Substituenten für die Alkyl-, Aryl- und Aralkylreste sind solche, wie sie vorher bei der Erläuterung des Restes R^{A-1} beschrieben worden sind.

Anschließend werden die ggfs. vorhandenen Schutzgruppen in an sich bekannter Weise wieder abgespalten.

Während nach Verfahrensvariante a) nur Insulinderivate der Formel III mit R³⁰ + R³¹ = OH (Des-B30-Insulinderivate) und R^{A-1} = Rest einer genetisch kodierbaren Aminosäure erhalten werden können,
ist nach Verfahrensvariante b) auch die Herstellung der Insulinderivate der Formel III mit den in der Legende zu Formel III unter b) genannten Resten für R³⁰ und R³¹ wie auch den physiologisch unbedenklichen organischen Resten mit bis zu 50 C-Atomen für R^{A-1} möglich.

Die Insulinderivate der Formel III sind in vivo voll wirksam, was im Hinblick auf die vorher erwähnte Literaturstelle P. Rösen et al außerordentlich überraschend ist; sie wirken sehr ähnlich den in der Patentanmeldung DE 38 44 211 beschriebenen AO-Arg-Insulinderivaten. Sie dienen daher - ebenso wie ihre physiologisch verträglichen Salze (wie z. B. die Na- oder NH₄-Salze) - in erster Linie als Wirkstoffe für pharmazeutische Zubereitungen zur Behandlung des Diabetes mellitus.

Erfindungsgegenstand ist daher auch eine pharmazeutische Zubereitung, die durch einen Gehalt an mindestens einem Insulinderivat der Formel III und/oder mindestens einem von dessen physiologisch verträglichen Salzen in gelöster, amorpher und/oder kristalliner - vorzugsweise in amorpher und/oder kristalliner - Form gekennzeichnet ist.

Für diese pharmazeutische Zubereitung bevorzugte Insulinderivate der Formel III sind
(A-1)-Thr-A0-Arg-Humaninsulin,
(A-1)Thr-A0-Arg-Schweineinsulin,
(A-1)Thr-A0-Arg-Kanincheninsulin,
(A-1)Thr-AO-Arg-Rinderinsulin und
(A-1)Thr-A0-Arg-Des-B30-Humaninsulin,
vorzugsweise nur (A-1)Thr-AO-Arg-Des-B30-Humaninsulin,
und deren physiologisch verträglichen Salze.

Die pharmazeutische Zubereitung ist vorzugsweise eine Lösung oder Suspension zu Injektionszwecken mit einem pH-Wert zwischen etwa 3,0 und 9,0, vorzugsweise zwischen etwa 5,0 und 8,5, welche
ein geeignetes Isotoniemittel,
ein geeignetes Konservierungsmittel und gegebenenfalls einen geeigneten Puffer,
sowie gegebenenfalls auch eine bestimmte Zinkionen-Konzentration oder ein anderes Depotprinzip wie z.B. Protaminsulfat,
alles natürlich in steriler wäßriger Lösung oder Suspension, enthält. Die Gesamtheit der Zubereitungsbestandteile außer dem Wirkstoff bildet den Zubereitungsträger.

Geeignete Isotoniemittel sind z.B. Glycerin, Glukose, Mannit, NaCl, Calcium- oder Magnesium-Verbindungen wie z.B. CaCl₂ MgCl₂ etc.

Geeignete Konservierungsmittel sind z.B. Phenol, m-Cresol, Benzylalkohol und/oder p-Hydroxybenzoesäureester.

Als Puffersubstanzen, insbesondere zur Einstellung eines pH-Wertes zwischen etwa 5,0 und 8,5, können z.B. Natriumacetat, Natriumcitrat, Natriumphosphat etc. verwendet werden. Ansonsten sind zur Einstellung des pH-Wertes auch physiologisch unbedenkliche verdünnte Säuren (typischerweise HCl) bzw. Laugen (typischerweise NaOH) geeignet.

Wenn die Zubereitung einen Zinkgehalt besitzt, ist ein solcher von etwa 1 µg bis 2 mg, insbesondere von etwa 5 µg bis 200 µg Zink/ml bevorzugt.

Zwecks Variation des Wirkungsprofils der erfindungsgemäßen Zubereitung können auch andere modifizierte (vgl. EP-B 132 769 und EP-B 132 770 sowie DE-Patentanmeldung DE 38 44 211 und/oder unmodifizierte Insuline, vorzugsweise Rinder-, Schweine-, Kaninchen- oder Humaninsulin, insbesondere Humaninsulin, zugemischt werden.

Bevorzugte Wirkstoffkonzentrationen sind solche entsprechend etwa 1 bis 15000, weiter bevorzugt etwa 5 bis 1000 und insbesondere etwa 40 bis 400 Internationale Einheiten/ml.

Die pharmazeutische Zubereitung wird dadurch hergestellt, daß man mindestens ein Insulinderivat der Formel III und/oder mindestens eines von dessen physiologisch verträglichen Salzen, gegebenenfalls zusammen mit anderen modifizierten und/oder unmodifizierten Insulin(derivat)en, mit einem physiologisch unbedenklichen Träger sowie gegebenenfalls mit geeigneten Zusatz- und Hilfsstoffen in eine geeignete Darreichungsform bringt.

Die Erfindung wird nun durch das folgende Beispiel näher erläutert.

Herstellung von (A-1)Thr-(A0)Arg-Des-B30-Humaninsulin nach Verfahrensvariante a)
200 mg des nach dem Verfahren gemäß DE-Patentanmeldung DE 38 21 159 hergestellten rekombinanten Proteins der Formel: 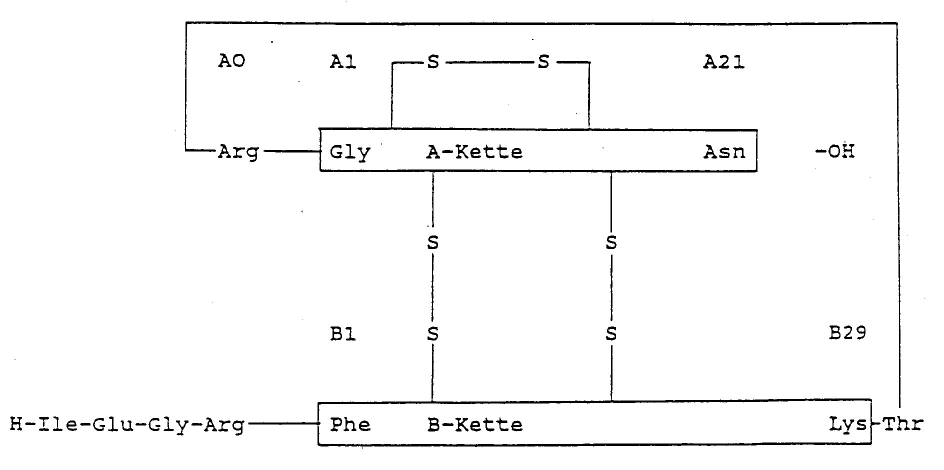
- d. i. eine Verbindung der Formel V, worin AS = Thr, x,z = O, Y = Arg und R = H-Ile-Glu-Gly -
wurden in 50 ml 20 mM Phosphatpuffer, pH 8,4, gelöst und mit 100 mcg Lysylendopeptidase während 30 Minuten bei 20°C verdaut. Anschließend spaltete man die Peptidsequenz Ile-Glu-Gly-Arg mit 100 mcg Trypsin ab. Nach 2 Stunden Reaktionszeit bei Raumtemperatur wurde der Ansatz durch Zugabe von Trifluoressigsäure abgestoppt und das Reaktionsprodukt auf Reversephase im 0,1 % Trifluoressigsäure/Acetonitril-System gereinigt. Es entstanden 51 mg (A-1)Thr-(AO)Arg-(B30)Des-Thr-Humaninsulin der Formel:

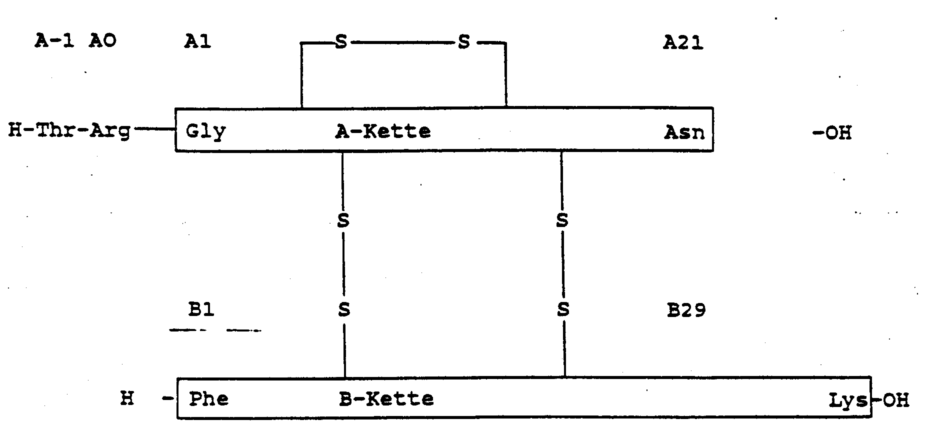

### Blutzuckersenkende Wirkung:

2,2 mg wasserfreies (A-1)Thr-(AO)Arg-(B30)Des-Thr-Humaninsulin wurden in 1,5 ml 0,9 %iger NaCl-Lösung aufgenommen. Als Vergleich und Standard-Substanz verwendete man 2,2 mg Humaninsulin, ebenfalls in 1,5 ml physiologischer Kochsalzlösung. Es wurden 5 nüchternen, männlichen Kaninchen mit Durchschnittsgewichten von 3,1 kg jeweils 6,25 microliter Probenlösung/kg Lebendgewicht intravenös verabreicht. 5 weiteren Tieren applizierte man entsprechend 6,25 microliter Standardlösung pro kg ebenfalls i.v. Nach verschiedenen Zeiten wurden Blutproben abgenommen und diese auf Blutzuckerkonzentrationen untersucht. Es wurden innerhalb der Fehlergrenzen folgende Meßergebnisse gefunden:

| | Blutglucose in % des Ausgangswertes nach | | | | |
|---|---|---|---|---|---|
| | 1/2 h | 1 h | 2 h | 3 h | 4 h |
| Standardlösung (Humaninsulin) | -28 | -27 | -18 | -11 | -6 |
| (A-1)Thr-(AO)Arg-(B30)Des-Thr-insulin | -31 | -31 | -20 | -3 | ±0 |

Aus den Zahlenwerten geht hervor, daß die blutzuckersenkende Wirkung des neuen Insulinderivats in der gleichen Größenordnung liegt wie diejenigen des (unmodifizierten) Humaninsulins.

Bei subcutaner Applikation einer Kristallsuspension wirkt das neue Insulinderivat verzögert.

## Patentansprüche (Patentansprüche für folgende(n) Vertragsstaat(en): AT, BE, CH, DE, DK, FR, GB, IT, LI, LU, NL, SE)

1. Insulinderivat der Formel III 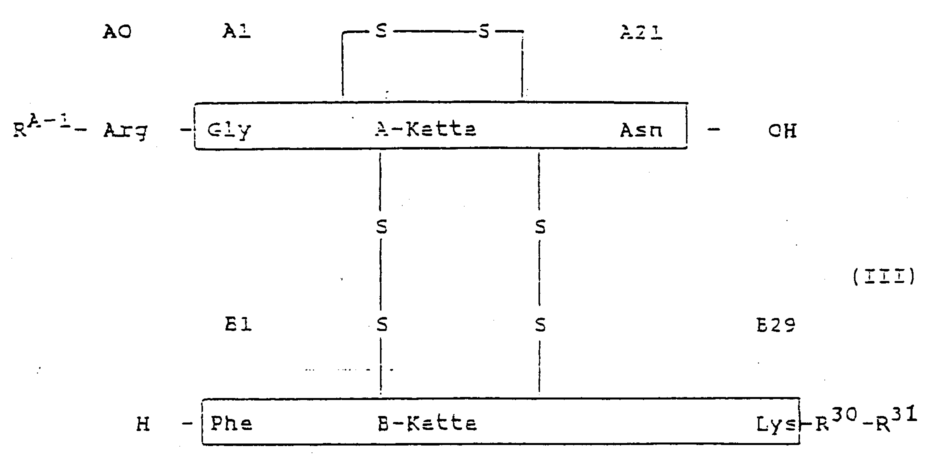 in welcher
a)
R³⁰ und R³¹ zusammen OH bedeuten oder
b)
R³⁰ bedeutet Rest einer neutralen, genetisch kodierbaren L-Aminosäure und
R³¹ bedeutet OH oder 1 bis 3 α-Aminosäuren, und
c)
R^{A-1} bedeutet Rest einer genetisch kodierbaren L-Aminosäure aus der Gruppe Thr, Ser oder Ala; sowie physiologisch verträgliche Salze des Insulinderivats der Formel III.

2. Insulinderivate und deren physiologisch verträgliche Salze nach Anspruch 1, dadurch gekennzeichnet, daß in Formel III
a)
R³⁰ + R³¹ = OH oder
b)
R³⁰ = Rest von Ala, Thr oder Ser, insbesondere von Thr, und R³¹ = OH, und
c)
R^{A-1} = Thr.

3. Insulinderivate und deren physiologisch verträgliche Salze nach Anspruch 1 oder 2,
dadurch gekennzeichnet, daß in Formel III
a) R³⁰ + R³¹ = OH und c) R^{A-1} = Thr.

4. Insulinderivate und deren physiologisch verträgliche Salze nach einem oder mehreren der Ansprüche 1 bis 3, dadurch gekennzeichnet, daß in Formel III die A-Kette und die B-Kette (B1 - B29) die Sequenzen des Human-, Schweine-, Kaninchen- oder Rinder-Insulins, vorzugsweise des Human-, Schweine- oder Kaninchen-Insulins sind.

5. Insulinderivate nach einem oder mehreren der Ansprüche 1 bis 4, gekennzeichnet durch einen isoelektrischen Punkt zwischen 5,5 und 9,0.

6. Verfahren zur Herstellung von Insulinderivaten der Formel III gemäß der Definition in einem oder mehreren der Ansprüche 1 bis 5,
dadurch gekennzeichnet, daß man
a) ein Insulinprodukt der Formel IV 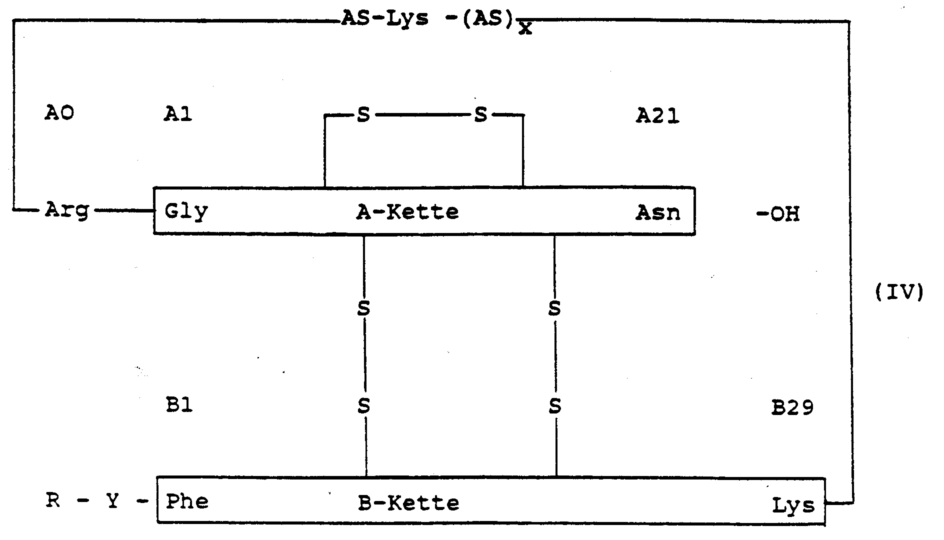 worin
AS = genetisch kodierbare Aminosäure(n),
x,z = unabhängig voneinander 0 oder ganze Zahlen von 1 - 50, wobei allerdings - falls x ≠ 0 - auch z ≠ 0 ist,
R = organischer Rest mit bis zu 50 C-Atomen, vorzugsweise ein Rest einer genetisch kodierbaren Aminosäure oder ein Peptidrest aus zwei oder mehreren gleichen oder verschiedenen genetisch kodierbaren Aminosäuren,
y = Lys oder Arg,
und die A- und B(1 bis 29)-Ketten vorzugsweise die Sequenzen des Human-, Schweine-, Kaninchen- oder Rinderinsulins, insbesondere des Human-, Schweine- oder Kanincheninsulins, aufweisen, mit Lysylendopeptidase in Kontakt bringt, wobei die Bindungen am C-terminalen Ende der Lysylreste gespalten werden, und - falls Y = Lys - die Verbindungen der Formel III mit R³⁰ + R³¹ zusammen = OH und R^{A-1} genetisch kodierbare Aminosäure erhalten werden, bzw. - falls Y = Arg - noch mit Trypsin oder 5 einer trypsinähnlichen Endopeptidase versetzt wird, wobei die Präaminosäuresequenz R-Arg von der B-Kette abgespalten wird und ebenfalls eine Verbindung der Formel III mit R³⁰ + R³¹ zusammen = OH und R^{A-1} = genetisch kodierbare Aminosäure entsteht, oder daß man
b) ein ggfs. geschütztes Insulinprodukt der Formel V 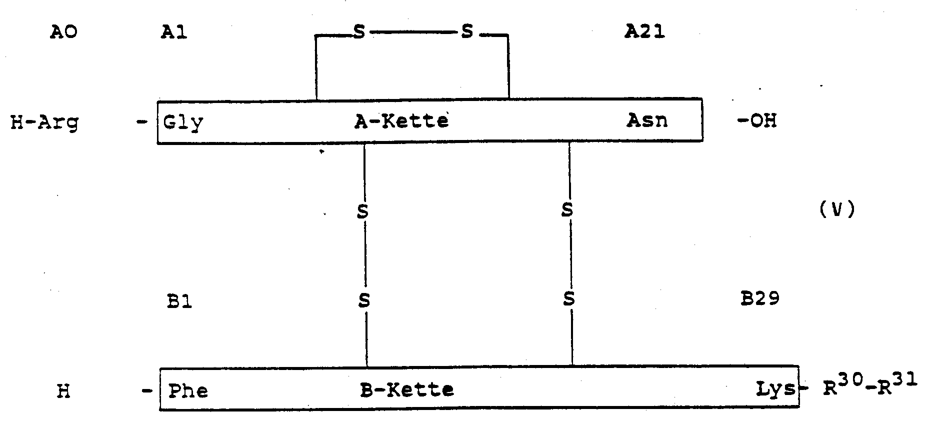 in welcher R³⁰ + R³¹ die gleiche Bedeutung wie in Formel III besitzen, am N-Terminus des (AO)-Arginins in an sich bekannter Weise mit ggfs. geschützten und/oder aktivierten, genetisch kodierbaren L-Aminosäuren, oder mit Donoren von physiologisch unbedenklichen organischen Resten mit bis zu 50 C-Atomen, vorzugsweise von - ggfs. substituierten - Alkyl-, Aryl- oder Alkarylresten, umsetzt und anschließend die vorhandenen Schutzgruppen in bekannter Weise abspaltet.

7. Verwendung der Insulinderivate und deren physiologisch verträglicher Salze gemäß einem oder mehreren der Ansprüche 1 bis 5 als Wirkstoffe für pharmazeutische Zubereitungen zur Behandlung von Diabetes mellitus.

8. Pharmazeutische Zubereitung, gekennzeichnet durch einen Gehalt an mindestens einem Insulinderivat der Formel III und/oder mindestens eines von deren physiologisch verträglichen Salzen gemäß der Definition in einem oder mehreren der Ansprüche 1 bis 5 in gelöster, amorpher und/oder kristalliner - vorzugsweiser in amorpher und/oder kristalliner - Form.

9. Pharmazeutische Zubereitung nach Anspruch 8, gekennzeichnet durch einen Gehalt an mindestens einer der folgenden - unter die Formel III fallenden - Insulinderivate:
(A-1)-Thr-A0-Arg-Humaninsulin,
(A-1)Thr-A0-Arg-Schweineinsulin,
(A-1)Thr-A0-Arg-Kanincheninsulin,
(A-1)Thr-AO-Arg-Rinderinsulin und
(A-1)Thr-A0-Arg-Des-B30-Humaninsulin,
vorzugsweise nur (A-1)Thr-AO-Arg-Des-B30-Humaninsulin, sowie deren physiologisch verträglichen Salze.

10. Pharmazeutische Zubereitung nach Anspruch 8 oder 9 als Injektionslösung bzw. -suspension mit einem pH-Wert zwischen etwa 3,0 und 9,0, vorzugsweise etwa 5,0 und 8,5.

11. Verfahren zur Herstellung einer pharmazeutischen Zubereitung gemäß einem oder mehreren der Ansprüche 8 bis 10, dadurch gekennzeichnet, daß man mindestens ein Insulinderivat der Formel III und/oder mindestens eines von deren physiologisch verträglichen Salzen, gegebenenfalls mit anderen modifizierten und/oder unmodifizierten Insulin(derivat)en, mit einem physiologisch annehmbaren Träger sowie gegebenenfalls geeigneten Zusatz- und/oder Hilfsstoffen in eine geeignete Darreichungsform bringt.

## Patentansprüche (Patentansprüche für folgende(n) Vertragsstaat(en): ES, GR)

1. Verfahren zur Herstellung von Insulinderivaten der Formel III 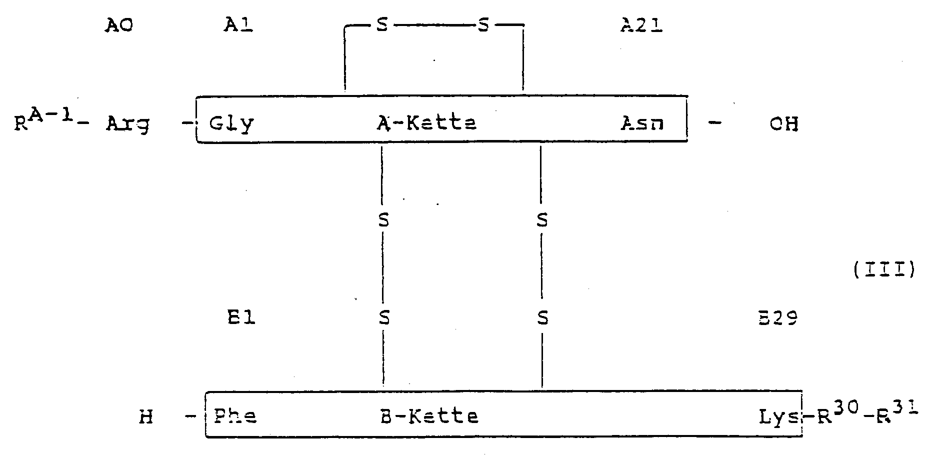 in welcher
a)
R³⁰ und R³¹ zusammen OH bedeuten oder
b)
R³⁰ bedeutet Rest einer neutralen, genetisch kodierbaren L-Aminosäure und
R³¹ bedeutet OH oder 1 bis 3 α-Aminosäuren, und
c)
R^{A-1} bedeutet Rest einer genetisch kodierbaren L-Aminosäure aus der Gruppe Thr, Ser oder Ala; sowie physiologisch verträgliche Salze des Insulinderivats der Formel III,
dadurch gekennzeichnet, daß man
a) ein Insulinprodukt der Formel IV 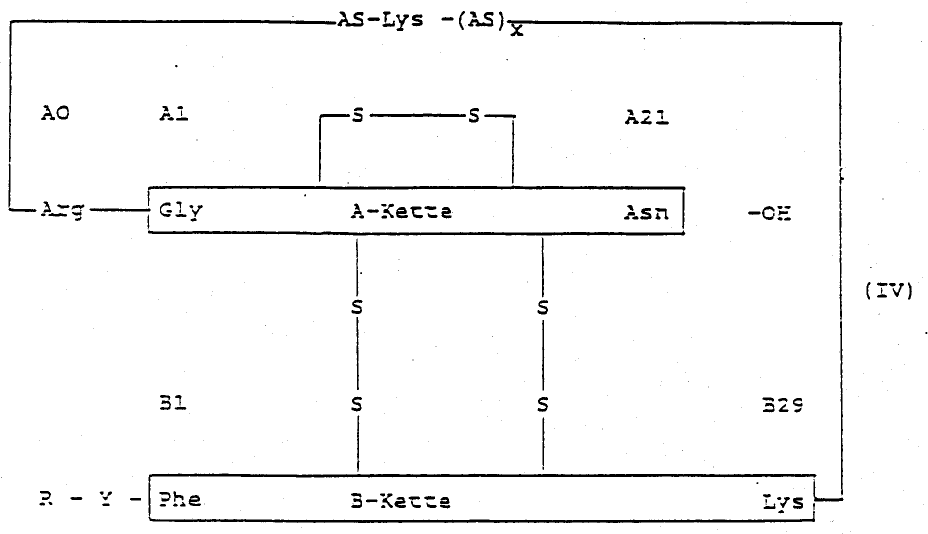 worin
AS = genetisch kodierbare Aminosäure(n),
x,z = unabhängig voneinander 0 oder ganze Zahlen von 1 - 50, wobei allerdings - falls x ≠ 0 - auch z ≠ 0 ist,
R = organischer Rest mit bis zu 50 C-Atomen, vorzugsweise ein Rest einer genetisch kodierbaren Aminosäure oder ein Peptidrest aus zwei oder mehreren gleichen oder verschiedenen genetisch kodierbaren Aminosäuren,
y = Lys oder Arg,
und die A- und B(1 bis 29)-Ketten vorzugsweise die Sequenzen des Human-, Schweine-, Kaninchen- oder Rinderinsulins, insbesondere des Human-, Schweine- oder Kanincheninsulins, aufweisen,
mit Lysylendopeptidase in Kontakt bringt, wobei die Bindungen am C-terminalen Ende der Lysylreste gespalten werden, und - falls Y = Lys - die Verbindungen der Formel III mit R³⁰ + R³¹ zusammen = OH und R^{A-1} = genetisch kodierbare Aminosäure erhalten werden, bzw. - falls Y = Arg - noch mit Trypsin oder 5 einer trypsinähnlichen Endopeptidase versetzt wird, wobei die Präaminosäureseguenz R-Arg von der B-Kette abgespalten wird und ebenfalls eine Verbindung der Formel III mit R³⁰ + R³¹ zusammen = OH und R^{A-1} = genetisch kodierbare Aminosäure entsteht, oder daß man
b) ein ggfs. geschütztes Insulinprodukt der Formel III 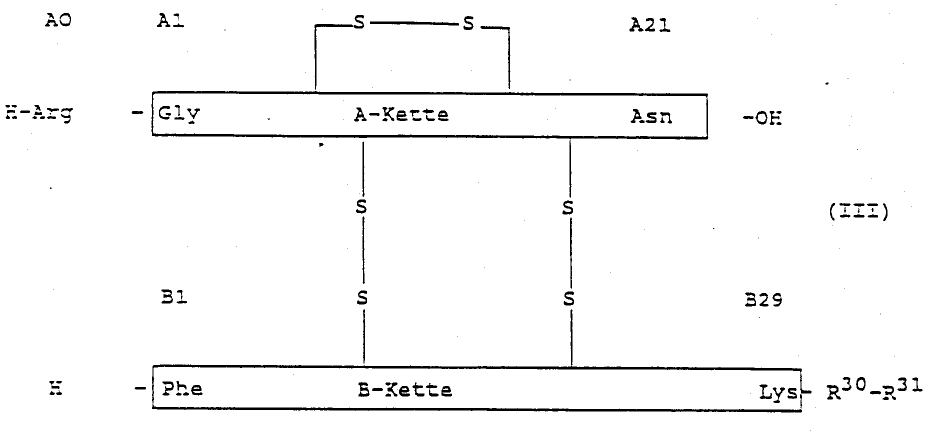 in welcher R³⁰ + R³¹ die gleiche Bedeutung wie in Formel III besitzen, am N-Terminus des (AO)-Arginins in an sich bekannter Weise mit ggfs. geschützten und/oder aktivierten, genetisch kodierbaren L-Aminosäuren, oder mit Donoren von physiologisch unbedenklichen organischen Resten mit bis zu 50 C-Atomen, vorzugsweise von - ggfs. substituierten - Alkyl-, Aryl- oder Alkarylresten, umsetzt und anschließend die vorhandenen Schutzgruppen in bekannter Weise abspaltet
und daß man gegebenenfalls die Endprodukte der Verfahrensvariante a oder b in deren physiologisch verträgliche Salze überführt.

2. Verfahren nach Anspruch 1, dadurch gekennzeichnet, daß man Insulinderivate der Formel III worin
a)
R³⁰ + R³¹ = OH oder
b)
R³⁰ = Rest von Ala, Thr oder Ser, insbesondere von Thr, und R³¹ = OH, und
c)
R^{A-1} = Thr
herstellt und gegebenenfalls in deren physiologisch verträgliche Salze überführt.

3. Verfahren nach Anspruch 1 oder 2, dadurch gekennzeichnet, daß man Insulinderivate der Formel III worin
a) R³⁰ + R³¹ = OH und c) R^{A-1} = Thr
herstellt und gegebenenfalls in deren physiologisch verträgliche Salze überführt.

4. Verfahren nach einem oder mehreren der Ansprüche 1 bis 3, dadurch gekennzeichnet, daß man Insulinderivate der Formel III, worin die A-Kette und die B-Kette (B1 - B29) die Sequenzen des Human-, Schweine-, Kaninchen- oder Rinder-Insulins, vorzugsweise des Human, Schweine- oder Kaninchen-Insulins sind, herstellt und gegebenenfalls in deren physiologisch verträglichen Salze überführt.

5. Verfahren nach einem oder mehreren der Ansprüche 1 bis 4, dadurch gekennzeichnet, daß man Insulinderivate der Formel III mit einem isoelektrischen Punkt zwischen 5,5 und 9,0 herstellt.

6. Verwendung der Insulinderivate gemäß der Definition in Anspruch 1 und deren physiologisch verträglichen Salze als Wirkstoffe für pharmazeutische Zubereitungen zur Behandlung von Diabetes mellitus.

7. Verfahren zur Herstellung einer pharmazeutischen Zubereitung, dadurch gekennzeichnet, daß man mindestens ein Insulinderivat der Formel III gemäß der Definition in Anspruch 1 und/oder mindestens eines von deren physiologisch verträglichen Salzen, gegebenenfalls mit anderen modifizierten und/oder unmodifizierten Insulin(derivat)en, mit einem physiologisch annehmbaren Träger sowie gegebenenfalls geeigneten Zusatz- und/oder Hilfsstoffen in eine geeignete Darreichungsform bringt.

8. Verfahren nach Anspruch 7, dadurch gekennzeichnet, daß man die Insulinderivate der Formel III und/oder deren physiologisch verträgliche Salze in gelöster, amorpher und/oder kristalliner - vorzugsweiser in amorpher und/oder kristalliner - Form einsetzt.

9. Verfahren nach Anspruch 7 und 8, dadurch gekennzeichnet, daß man mindestens eine der folgenden - unter die Formel III fallenden - Insulinderivate einsetzt:
(A-1)-Thr-A0-Arg-Humaninsulin,
(A-1)Thr-A0-Arg-Schweineinsulin,
(A-1)Thr-A0-Arg-Kanincheninsulin,
(A-1)Thr-AO-Arg-Rinderinsulin und
(A-1)Thr-A0-Arg-Des-B30-Humaninsulin,
vorzugsweise nur (A-1)Thr-AO-Arg-Des-B30-Humaninsulin, sowie deren physiologisch verträglichen Salze.

10. Verfahren nach einem oder mehreren der Ansprüche 7 bis 9, dadurch gekennzeichnet, daß man die pharmazeutische Zubereitung als Injektionslösung bzw. -suspension mit einem pH-Wert zwischen etwa 3,0 und 9,0, vorzugsweise etwa 5,0 und 8,5 herstellt.

## Claims (Claims for the following Contracting State(s): AT, BE, CH, DE, DK, FR, GB, IT, LI, LU, NL, SE)

1. An insulin derivative of the formula III 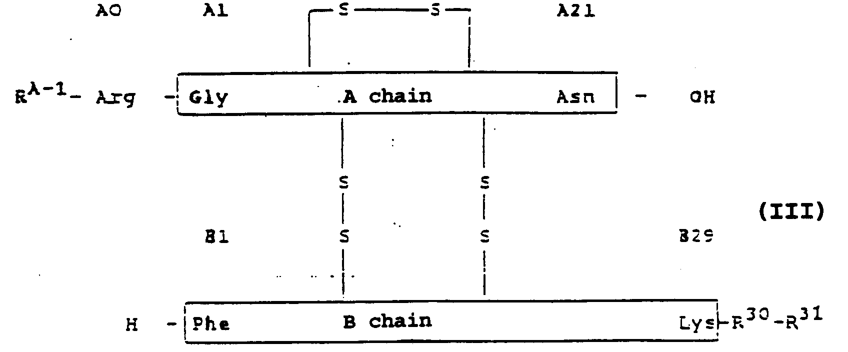 in which
a)
R³⁰ and R³¹ together are OH or
b)
R³⁰ is a radical of a neutral, genetically encodable L-amino acid and
R³¹ is OH or 1 to 3 α-amino acids, and
c)
R^{A-1} is a radical of a genetically encodable L-amino acid from the group consisting of Thr, Ser or Ala; or a physiologically tolerable salt of the insulin derivative of the formula III.

2. The insulin derivative or its physiologically tolerable salt as clawed in claim 1, wherein, in formula III,
a)
R³⁰ + R³¹ = OH or
b)
R³⁰ = a radical of Ala, Thr or Ser, in particular of Thr, and R³¹ = OH, and
c)
R^{A-1} = Thr.

3. The insulin derivative or its physiologically tolerable salt as claimed in claim 1 or 2, wherein, in formula III,
a) R³⁰ + R³¹ = OH and c) R^{A-1} = Thr.

4. The insulin derivative or its physiologically tolerable salt as claimed in one or more of claims 1 to 3, wherein, in formula III, the A chain and the B chain (B1 - B29) are the sequences of human, porcine, rabbit or bovine insulin, preferably of human, porcine or rabbit insulin.

5. The insulin derivative as claimed in one or more of claims 1 to 4, which has an isoelectric point between 5.5 and 9.0.

6. A process for the production of an insulin derivative of the formula III as claimed in the definition in one or more of claims 1 to 5, which comprises
a) bringing an insulin product of the formula IV 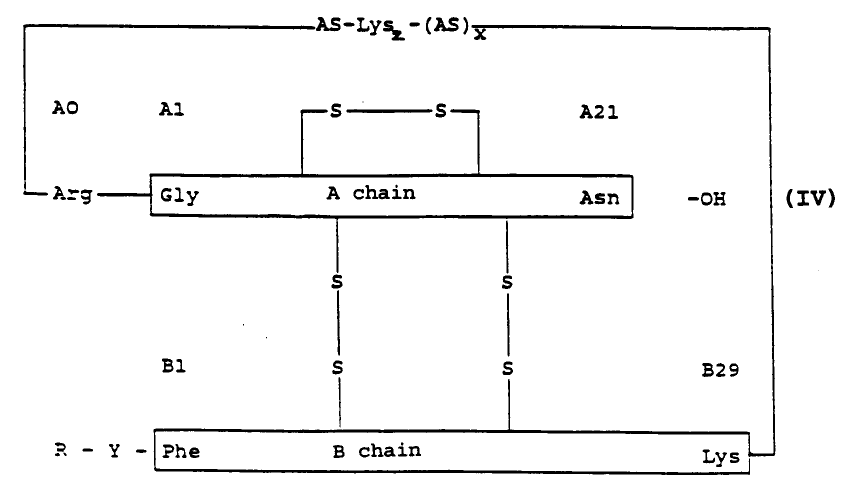 in which
AS = genetically encodable amino acid(s),
x, z = independently of one another 0 or integers from 1 - 50, where, however, - if x ≠ 0 - also z ≠ 0,
R = an organic radical having up to 50 carbon atoms, preferably a radical of a genetically encodable amino acid or a peptide radical formed from two or more identical or different genetically encodable amino acids,
y = Lys or Arg,
and the A and B(1 to 29) chains preferably have the sequences of human, porcine, rabbit or bovine insulin, in particular of human, porcine or rabbit insulin,
into contact with lysyl endopeptidase, the bonds at the C-terminal end of the lysyl radicals being cleaved, and - if Y = Lys - the compounds of the formula III where R³⁰ + R³¹ together - OH and R^{A-1} = genetically encodable amino acid being obtained, or - if Y = Arg - trypsin or a trypsin-like endopeptidase also being added, the preamino acid sequence R-Arg being cleaved from the B chain and a compound of the formula III where R³⁰ + R³¹ together - OH and R^{A-1} = genetically encodable amino acid likewise being formed, or
b) reacting an optionally protected insulin product of the formula V 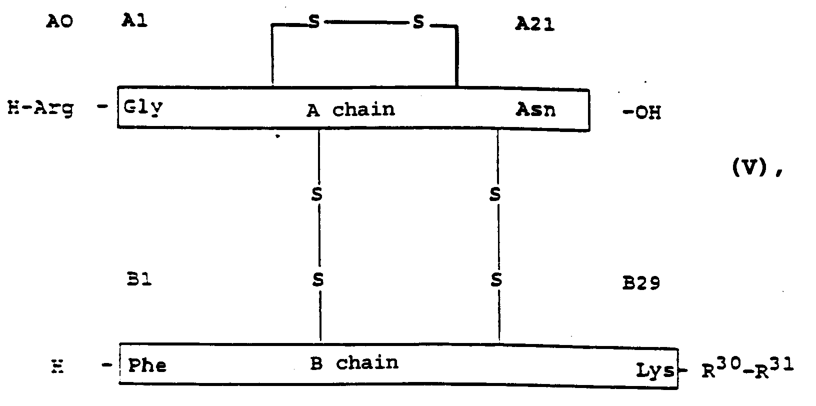 in which R³⁰ + R³¹ have the same meaning as in formula III, at the N-terminus of the (AO) arginine in a manner known per se with optionally protected and/or activated, genetically encodable L-amino acids, or with donors of physiologically acceptable organic radicals having up to 50 carbon atoms, preferably of - optionally substituted - alkyl, aryl or alkaryl radicals and then removing the protective groups present in a known manner.

7. The use of the insulin derivative or its physiologically tolerable salt as claimed in one or more of claims 1 to 5 as active compound for pharmaceutical preparations for the treatment of diabetes mellitus.

8. A pharmaceutical preparation which comprises at least one insulin derivative of the formula III and/or at least one of its physiologically tolerable salts as claimed in the definition in one or more of claims 1 to 5 in dissolved, amorphous and/or crystalline form - preferably in amorphous and/or crystalline form.

9. The pharmaceutical preparation as claimed in claim 8, which comprises at least one of the following insulin derivatives - coming under the formula III:
(A-1)ThrAO-Arg human insulin,
(A-1)Thr-AO-Arg porcine insulin,
(A-1)Thr-AO-Arg rabbit insulin,
(A-1)Thr-AO-Arg bovine insulin and
(A-1)Thr-AO-Arg-Des-B30 human insulin,
preferably only (A-1)Thr-AO-Arg-Des-B30 human insulin,
and their physiologically tolerable salts.

10. The pharmaceutical preparation as claimed in claim 8 or 9 as an injection solution or suspension having a pH between about 3.0 and 9.0, preferably about 5.0 and 8.5.

11. A process for the production of a pharmaceutical preparation as claimed in one or more of claims 8 to 10, which comprises bringing at least one insulin derivative of the formula III and/or at least one of its physiologically tolerable salts, if desired with other modified and/or unmodified insulins or insulin derivatives, into a suitable presentation form using a physiologically acceptable excipient and, if desired, suitable additives and/or auxiliaries.

## Claims (Claims for the following Contracting State(s): ES, GR)

1. A process for the production of an insulin derivative of the formula III 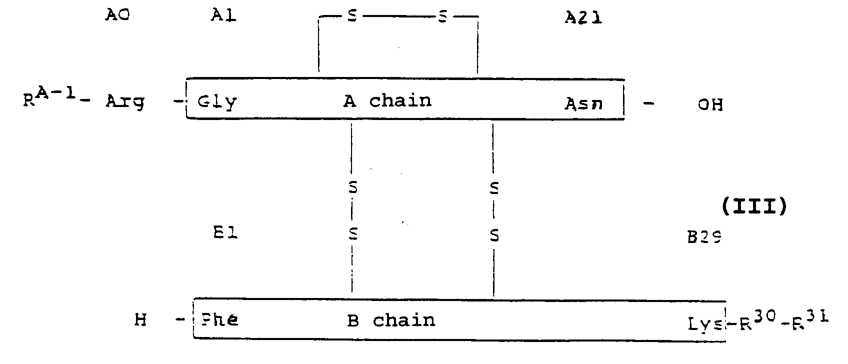 in which
a)
R³⁰ and R³¹ together are OH or
b)
R³⁰ is a radical of a neutral, genetically encodable L-amino acid and
R³¹ is OH or 1 to 3 α-amino acids and
c)
R^{A-1} is a radical of a genetically encodable L-amino acid from the group consisting of Thr, Ser or Ala; or a physiologically tolerable salt of the insulin derivative of the formula III,
which comprises
a) bringing an insulin product of the formula IV 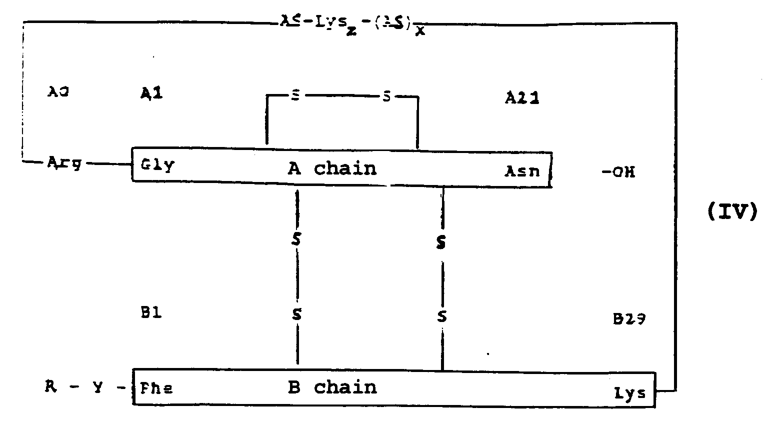 in which
AS = genetically encodable amino acid(s),
x, z = independently of one another 0 or integers from 1 - 50, where, however, - if x ≠ 0 - also z ≠ 0,
R = an organic radical having up to 50 carbon atoms, preferably a radical of a genetically encodable amino acid or a peptide radical formed from two or more identical or different genetically encodable amino acids,
y = Lys or Arg,
and the A and B(1 to 29) chains preferably have the sequences of human, porcine, rabbit or bovine insulin, in particular of human, porcine or rabbit insulin,
into contact with lysyl endopeptidase, the bonds at the C-terminal end of the lysyl radicals being cleaved, and - if Y = Lys - the compounds of the formula III where R³⁰ + R³¹ together = OH and R^{A-1} = genetically encodable amino acid being obtained, or - if Y = Arg - trypsin or a trypsin-like endopeptidase also being added, the proamino acid sequence R-Arg being cleaved from the B chain and a compound of the formula III where R³⁰ + R³¹ together = OH and R^{A-1} = genetically encodable amino acid likewise being formed, or
b) reacting an optionally protected insulin product of the formula V 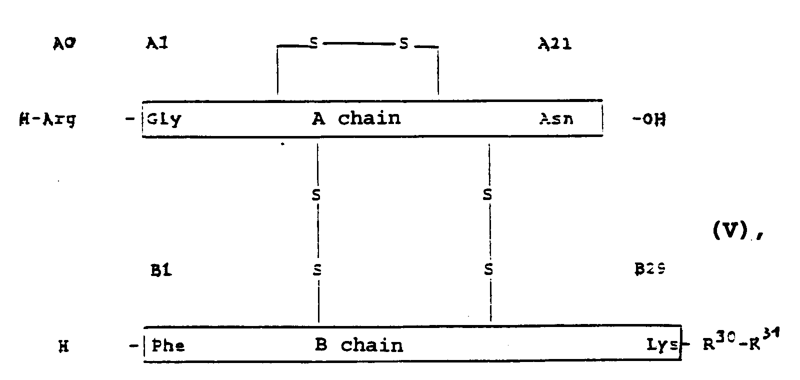 in which R³⁰ + R³¹ have the same meaning as in formula III, at the N-terminus of the (AO) arginine in a manner known per se with optionally protected and/or activated, genetically encodable L-amino acids, or with donors of physiologically acceptable organic radicals having up to 50 carbon atoms, preferably of - optionally substituted - alkyl, aryl or alkaryl radicals and then removing the protective groups present in a known manner
and optionally converting the end products of process variant a or b into their physiologically tolerable salts.

2. The process as claimed in claim 1, wherein an insulin derivative of the formula III is prepared in which
a)
R³⁰ + R³¹ = OH or
b)
R³⁰ = a radical of Ala, Thr or Ser, in particular of Thr, and R³¹ = OH, and
c)
R^{A-1} = Thr
and optionally converted into its physiologically tolerable salt.

3. The process as claimed in claim 1 or 2, wherein an insulin derivative of the formula III is prepared in which a) R³⁰ + R³¹ = OH and c) R^{A-1} = Thr and is optionally converted into its physiologically tolerable salt.

4. The process as claimed in one or more of claims 1 to 3, wherein an insulin derivative of the formula III is prepared in which the A chain and the B chain (B1 - B29) are the sequences of human, porcine, rabbit or bovine insulin, preferably of human, porcine or rabbit insulin, and is optionally converted into its physiologically tolerable salt.

5. The process as claimed in one or more of claims 1 to 4, wherein an insulin derivative of the formula III is prepared which has an isoelectric point between 5.5 and 9.0.

6. The use of the insulin derivative as claimed in the definition in claim 1 or its physiologically tolerable salt as active compound for pharmaceutical preparations for the treatment of diabetes mellitus.

7. A process for the production of a pharmaceutical preparation, which comprises bringing at least one insulin derivative of the formula III as claimed in the definition in claim 1 and/or at least one of its physiologically tolerable salts, if desired with other modified and/or unmodified insulins or insulin derivatives, into a suitable presentation form using a physiologically acceptable excipient and, if desired, suitable additives and/or auxiliaries.

8. The process as claimed in claim 7, wherein an insulin derivative of the formula III and/or its physiologically tolerable salt are employed in dissolved, amorphous and/or crystalline form - preferably in amorphous and/or crystalline form.

9. The process as claimed in claim 7 and 8, wherein at least one of the following insulin derivatives coming under the formula III - is employed:
(A-1)Thr-AO-Arg human insulin,
(A-1)Thr-AO-Arg porcine insulin,
(A-1)Thr-AO-Arg rabbit insulin,
(A-1)Thr-AO-Arg bovine insulin and
(A-1)Thr-AO-Arg-Des-B30 human insulin,
preferably only (A-1)Thr-AO-Arg-Des-B30 human insulin,
and their physiologically tolerable salts.

10. The process as claimed in one or more of claims 7 to 9, wherein the pharmaceutical preparation is produced as an injection solution or suspension having a pH between about 3.0 and 9.0, preferably about 5.0 and 8.5.

## Revendications (Revendications pour l'(les) Etat(s) contractant(s) suivant(s): AT, BE, CH, DE, DK, FR, GB, IT, LI, LU, NL, SE)

1. Dérivé de l'insuline de formule III 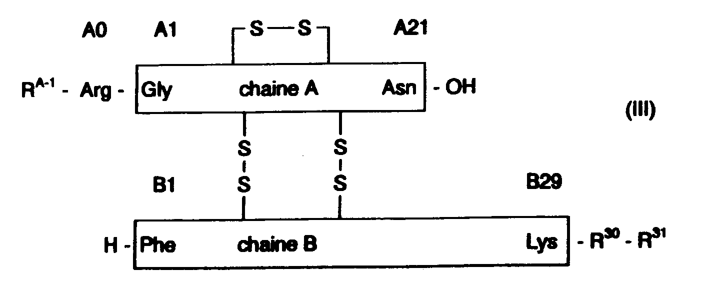 où
a)
R³⁰ et R³¹ signifient ensemble OH ou
b)
R³⁰ signifie un reste d'un L-aminoacide neutre codable génétiquement et
R³¹ signifie OH ou 1 à 3 α-aminoacides, et
c)
R^{A-1} signifie un reste d' un L-aminoacide neutre codable génétiquement du groupe Thr, Ser ou Ala; ainsi que les sels physiologiquement acceptables du dérivé de l'insuline de formule III.

2. Dérivés de l'insuline et leurs sels physiologiquement acceptables selon la revendication 1, caractérisés en ce que, dans la formule III,
a)
R³⁰ + R³¹ = OH ou
b)
R³⁰ = reste d'Ala, Thr ou Ser, en particulier de Thr, et R³¹ = OH, et
c)
R^{A-1} = Thr.

3. Dérivés de l'insuline et leurs sels physiologiquement acceptables selon la revendication 1 ou 2, caractérisés en ce que, dans la formule III
a) R³⁰ + R³¹ = OH et c) R^{A-1} = Thr.

4. Dérives de l'insuline et leurs sels physiologiquement acceptables selon une ou plusieurs des revendications 1 à 3, caractérisés en ce que, dans la formule III, la chaîne A et la chaîne B(B1 - B29) sont les séquences de l'insuline humaine, de porc, de lapin ou de boeuf, de préférence de l'insuline humaine, de porc ou de lapin.

5. Dérivés de l'insuline selon une ou plusieurs des revendications 1 à 4, caractérisés par un point isoélectrique compris entre 5,5 et 9,0.

6. Procédé pour la préparation des dérivés de l'insuline de formule III selon la définition énoncée dans une ou plusieurs des revendications 1 à 5, caractérisé en ce que
a) un produit insulinique de formule IV 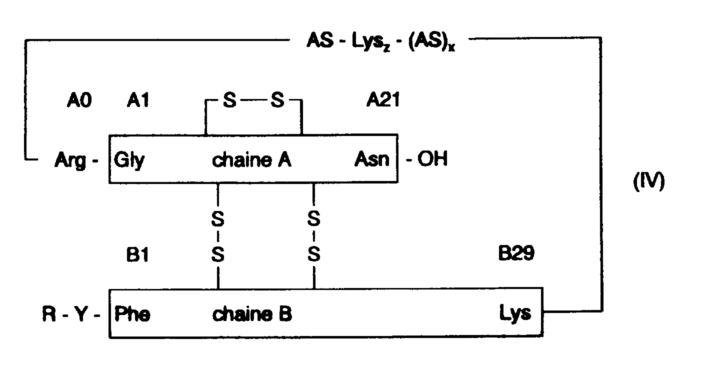 où
AS = aminoacide(s) codables(s) génétiquement,
x, z indépendants l'un de l'autre = 0 ou entier de 1 à 50, mais si x ≠ 0, alors z ≠ 0 également,
R = groupe organique comprenant jusqu'à 50 atomes de C, de préférence un reste d' aminoacide génétiquement codable ou un reste peptidique constitué de deux ou de plusieurs aminoacides génétiquement codables, identiques ou différents,
Y = Lys ou Arg,
et les chaînes A et B(1 à 29) représentent de préférence les séquences de l'insuline humaine, de porc, de lapin ou de boeuf, en particulier de l'insuline humaine, de porc ou de lapin,
est mis contact avec une lysylendopeptidase, ce qui provoque la scission des liaisons à l'extrémité C-terminale des restes lysyle, et - dans le cas où Y = Lys - les composés de formule III où R³⁰ + R³¹ ensemble représentent OH et R^{A-1} est un acide aminé génétiquement codable sont obtenus, ou bien - dans le cas où Y = Arg - on les met encore en présence de trypsine ou d'une endopeptidase semblable à la trypsine, ce qui provoque la scission de la séquence pré-aminoacide R-Arg de la chaîne B et on obtient également un composé de formule III où R³⁰ + R³¹ ensemble représentent OH et R^{A-1} est un aminoacide génétiquement codable, ou bien en ce que
b) un produit insulinique éventuellement protégé, de formule V, 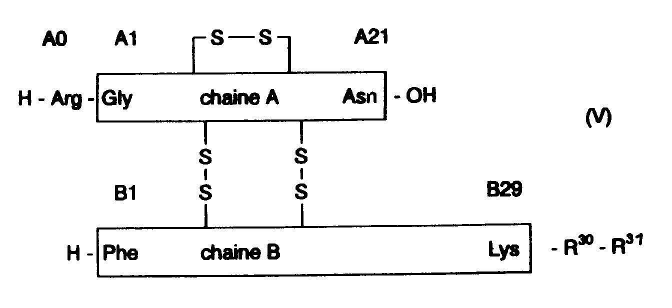 où R³⁰ et R³¹ ont la même signification que dans la formule III, réagit à l'extrémité N-terminale de l'arginine (A0) de façon connue avec un L-aminoacide génétiquement codable, éventuellement protégé et/ou activé, ou des donneurs de groupes organiques sans inconvénients physiologiques, comprenant jusqu'à 50 atomes de C, de préférence les groupes alkyle, aryle ou alkaryle éventuellement substitués, et est finalement déprotégé des groupes protecteurs éventuellement présents à l'aide de méthodes connues.

7. Utilisation des dérivés de l'insuline et de leurs sels physiologiquement acceptables selon une ou plusieurs des revendications 1 à 5, en tant que principe actif dans des compositions pharmaceutiques pour le traitement du diabète sucré.

8. Composition pharmaceutique caractérisée par sa teneur en au moins un des dérivés de l'insuline de formule III et/ou au moins un de ses sels physiologiquement acceptables selon la définition donnée dans une ou plusieurs des revendications 1 à 5, sous forme solubilisée, amorphe et/ou cristalline, de préférence amorphe et/ou cristalline.

9. Composition pharmaceutique selon la revendication 8, caractérisée par sa teneur en au moins un des dérivés de l'insuline suivants - correspondant à la formule III -
(A-1)Thr-A0-Arg-insuline humaine,
(A-1)Thr-A0-Arg-insuline de porc,
(A-1)Thr-A0-Arg-insuline de lapin,
(A-1)Thr-A0-Arg-insuline de boeuf et
(A-1)Thr-A0-Arg-Des-B30-insuline humaine,
de préférence seulement la (A-1 )Thr-A0-Arg-Des-B30-insuline humaine et ses sels physiologiquement acceptables.

10. Composition pharmaceutique selon la revendication 8 ou 9 sous forme de solution ou suspension injectable de pH compris entre environ 3,0 et 9,0, de préférence entre environ 5,0 et 8,5.

11. Procédé pour la préparation d'une composition pharmaceutique selon une ou plusieurs des revendications 8 à 10, caractérisé en ce que l'on met sous une forme appropriée de présentation, au moins un dérivé de l'insuline de formule III et/ou au moins un de ses sels physiologiquement acceptables, éventuellement avec d'autres (dérivés de l') insuline(s), modifié(e)s et/ou non modifié(e)s, avec un véhicule physiologiquement acceptable ainsi qu'éventuellement des substances additionnelles et/ou auxiliaires appropriées.

## Revendications (Revendications pour l'(les) Etat(s) contractant(s) suivant(s): ES, GR)

1. Procédé pour la préparation des dérivés de l'insuline de formule III 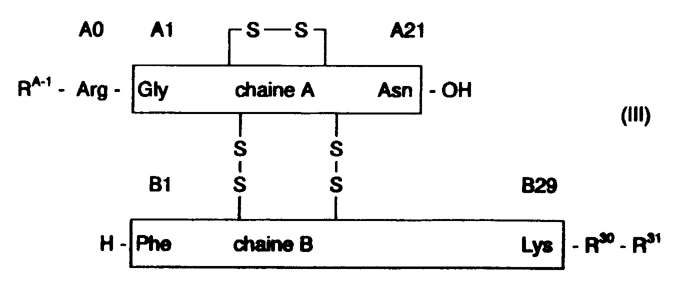 où
a)
R³⁰ et R³¹ signifient ensemble OH ou
b)
R³⁰ signifie un reste d' un L-aminoacide neutre codable génétiquement et
R³¹ signifie OH ou 1 à 3 α-aminoacides, et
c)
R^{A-1} signifie un reste d' un L-aminoacide neutre codable génétiquement du groupe Thr, Ser ou Ala; ainsi que les sels physiologiquement acceptables du dérivé de l'insuline de formule III,
caractérisé en ce que
a) un produit insulinique de formule IV 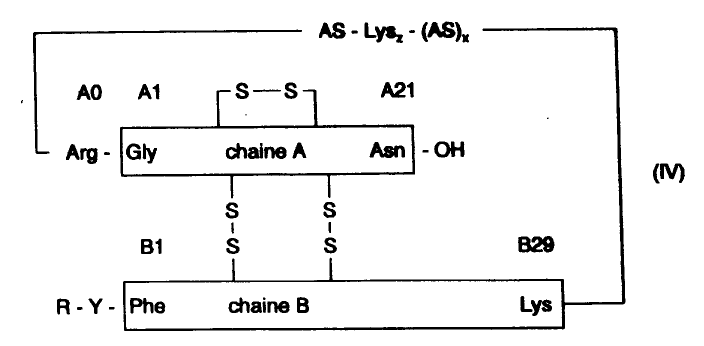 où
AS = aminoacide(s) codables(s) génétiquement,
x, z indépendants l'un de l'autre = 0 ou entier de 1 à 50, mais si x ≠ 0, alors z ≠ 0 également,
R = groupe organique comprenant jusqu'à 50 atomes de C, de préférence un reste d'un acide aminé génétiquement codable ou un reste peptidique constitué de deux ou de plusieurs aminoacides génétiquement codables, identiques ou différents,
Y = Lys ou Arg,
et les chaînes A et B(1 à 29) représentent de preference les séquences de l'insuline humaine, de porc, de lapin ou de boeuf, en particulier de l'insuline humaine, de porc ou de lapin,
est mis contact avec une lysylendopeptidase, ce qui provoque la scission des liaisons à l'extrémité C-terminale des restes lysyle, et - dans le cas où Y = Lys - les composés de formule III où R³⁰ + R³¹ ensemble représentent OH et R^{A-1} est un aminoacide génétiquement codable sont obtenus, ou bien - dans le cas où Y = Arg - on les met encore en présence de trypsine ou d'une endopeptidase semblable à la trypsine, ce qui provoque la scission de la séquence pré-aminoacide R-Arg de la chaîne B et on obtient également un composé de formule III où R³⁰ + R³¹ ensemble représentent OH et R^{A-1} est un aminoacide génétiquement codable, ou bien en ce que
b) un produit insulinique éventuellement protégé, de formule V, 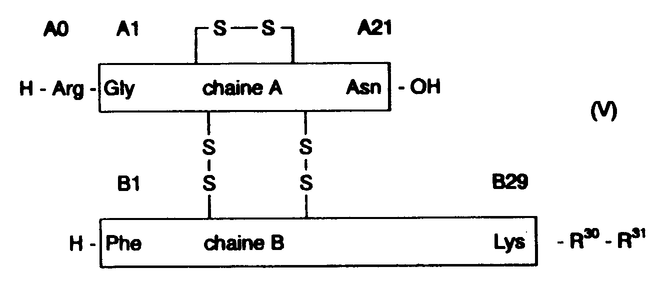 où R³⁰ et R³¹ ont la même signification que dans la formule III, réagit à l'extrémité N-terminale de l'arginine (A0) de façon connue avec un L-aminoacide génétiquement codable, éventuellement protégé et/ou activé, ou des donneurs de groupes organiques sans inconvénients physiologiques, comprenant jusqu'à 50 atomes de C, de préférence les groupes alkyle, aryle ou alkaryle éventuellement substitués, et est finalement déprotégé des groupes protecteurs éventuellement présents à l'aide de méthodes connues
et que l'on transforme éventuellement les produits finals des deux variantes a ou b du procédé en leurs sels physiologiquement acceptables.

2. Procédé selon la revendication 1 caractérisé en ce que l'on fabrique des dérivés de l'insuline de formule III où
a)
R³⁰ + R³¹ ensemble = OH ou
b)
R³⁰ = un reste d'Ala, Thr ou Ser, en particulier Thr, et R³¹ = OH, et
c)
R^{A-1} = Thr
et que l'on transforme éventuellement en leurs sels physiologiquement acceptables.

3. Procédé selon la revendication 1 ou 2, caractérisé en ce que l'on fabrique des dérivés de l'insuline de formule III où
a) R³⁰ + R³¹ = OH et c) R^{A-1} = Thr
et que l'on transforme éventuellement en leurs sels physiologiquement acceptables.

4. Procédé selon une ou plusieurs des revendications 1 à 3, caractérisé en ce que l'on fabrique des dérivés de l'insuline de formule III dont la chaîne A et la chaîne B(B1 - B29) sont les séquences de l'insuline humaine, de porc, de lapin ou de boeuf, de préférence de l'insuline humaine, de porc ou de lapin, et que l'on transforme éventuellement en leurs sels physiologiquement acceptables.

5. Procédé selon une ou plusieurs des revendications 1 à 4, caractérisé en ce que l'on fabrique des dérivés de l'insuline de formule III dont le point isoélectrique est compris entre 5,5 et 9,0.

6. Utilisation des dérivés de l'insuline selon la définition énoncée dans la revendication 1 et de leurs sels physiologiquement acceptables en tant que principe actif dans des compositions pharmaceutiques pour le traitement du diabéte sucré.

7. Procédé pour la préparation d'une composition pharmaceutique, caractérisé en ce que l'on met sous une forme appropriée de présentation, au moins un dérivé de l'insuline de formule III selon la définition énoncée dans la revendication 1 et/ou au moins un de ses sels physiologiquement acceptables, éventuellement avec d'autres (dérivés de l')insuline(s) modifié(e)s et/ou non modifié(e)s, avec un véhicule physiologiquement acceptable ainsi qu'éventuellement des substances additionnelles et/ou auxiliaires appropriées.

8. Procédé selon la revendication 7, caractérisé en ce que l'on utilise les dérivés de l'insuline de formule III et/ou leurs sels physiologiquement acceptables sous forme solubilisée, amorphe et/ou cristalline, de préférence amorphe et/ou cristalline.

9. Procédé selon les revendications 7 et 8, caractérisé par l'utilisation d'au moins un des dérivés de l'insuline suivants - correspondants à la formule III -
(A-1)Thr-A0-Arg-insuline humaine,
(A-1)Thr-A0-Arg-insuline de porc,
(A-1)Thr-A0-Arg-insuline de lapin,
(A-1)Thr-A0-Arg-insuline de boeuf et
(A-1)Thr-A0-Arg-Des-B30-insuline humaine,
de préférence seulement la (A-1)Thr-A0-Arg-Des-B30-insuline humaine et ses sels physiologiquement acceptables.

10. Procédé selon une ou plusieurs des revendications 7 à 9, caractérisé en ce que l'on fabrique la composition pharmaceutique sous forme de solution ou de suspension injectable avec un pH compris entre environ 3,0 et 9,0, de préférence entre environ 5,0 et 8,5.
